# EUROPEAN PATENT SPECIFICATION

(11) **EP 4 260 335 B1**
(45) Date of publication and mention of the grant of the patent: **09.12.2026**
(21) Application number: 21836410.7
(22) Date of filing: 09.12.2021
(51) Int. Cl.: G16H 30/20, G16H 30/40

(54) **ACTIONABLE VISUALIZATION BY OVERLAYING HISTORICAL DATA ON A REAL-TIME IMAGE ACQUISITION WORKFLOW OVERVIEW**
UMSETZBARE VISUALISIERUNG DURCH ÜBERLAGERUNG VON HISTORISCHEN DATEN MIT EINER ÜBERSICHT ÜBER DEN BILDAUFNAHME-WORKFLOW IN ECHTZEIT
UNE VISUALISATION EXPLOITABLE EN SUPERPOSANT DES DONNÉES HISTORIQUES SUR UN APERÇU DU FLUX D'ACQUISITION D'IMAGES EN TEMPS RÉEL

(30) Priority: 11.12.2020 US 202063124107 P
(43) Date of publication of application: 18.10.2023
(73) Proprietor: Koninklijke Philips N.V., 5656 AE Eindhoven (NL)
(72) Inventor: KOKER, Ekin, 5656 AG Eindhoven (NL); TELLIS, Ranjith Naveen, 5656 AG Eindhoven (NL); CHADUVULA, Siva Chaitanya, 5656 AG Eindhoven (NL); CHEN, Xiuye, 5656 AG Eindhoven (NL); STAROBINETS, Olga, 5656 AG Eindhoven (NL); DALAL, Sandeep Madhukar, 5656 AG Eindhoven (NL); QIAN, Yuechen, 5656 AG Eindhoven (NL)
(74) Representative: Philips Intellectual Property & Standards
(86) International application number: PCT/EP2021/084910
(87) International publication number: WO 2022/122873

(56) References cited:
- EP-A2- 1 239 396
- US-A1- 2019 156 484
- US-A1- 2019 261 938
- US-B2- 9 047 661

## Description

The following relates generally to the imaging arts, remote imaging assistance arts, remote imaging examination monitoring arts, imaging data analytics arts, and related arts.

### BACKGROUND

The demand for high quality medical imaging by techniques such as magnetic resonance imaging (MRI), transmission computed tomography (CT), positron emission tomography (PET), and other medical imaging modalities is high, and is expected to increase with an aging population in many countries and other factors such as improved imaging system capabilities and improved techniques for generating actionable clinical findings from medical images. The increasing problem of getting highly qualified staff (sometimes referred to as imaging technicians or technologists) for performing complex medical imaging examinations has driven the concept of bundling medical expertise in remote service centers. The basic idea is to provide virtual availability of Senior Technologists as on-call experts in case a (local, on-site) technologist or operator performing a medical imaging examination needs assistance with a scheduled examination or runs into unexpected difficulties. In either case, the remote expert would remotely assist the on-site operator by receiving real-time views of the situation by way of screen mirroring of the display of the medical imaging device controller and optionally other information feeds such as one or more video feeds of the imaging bay. The remote expert typically would not directly operate the medical imaging device, but would provide advice or other input for assisting the local technologist by way of telephonic or videoconferencing communication.

To make such a remote service center commercially viable, it would be advantageous to enable the remote expert to concurrently assist (or be on call to assist) a number of different local technologists performing possibly concurrent medical imaging examinations. The local technologists may be located in a single hospital, or may be distributed among several hospitals in the same geographic area (e.g. a single city) or across a larger geographical area (e.g., spread across several states or even different countries). Preferably, the remote service center would be able to connect the expert to imaging systems of different models and/or manufactured by different vendors, since many hospitals maintain a heterogeneous fleet of imaging systems. This can be achieved by screen sharing or screen mirroring technologies that provide the remote expert a real-time copy of the imaging device controller display, optionally along with video cameras to provide views of the imaging bay and, optionally, the interior of the bore or other examination region of the imaging device. Such scalability can enable many local operators to benefit from the assistance of a single highly qualified remote expert (or small group of highly qualified remote experts) in a cost-effective manner.

The remote expert is assumed to have experience and expertise with the different user interfaces of the different medical imaging systems and vendors for which the expert is qualified to provide assistance. When providing (potentially simultaneous) assistance to multiple imaging bays, the expert is expected to rapidly switch between the screen views of the different imaging systems to extract the required pieces of information for quickly assessing the situation in each imaging bay. This is challenging as required pieces of information may be differently located on differently designed user interfaces, and some information that would be useful to the remote expert may not be shown at all on the current display. For example, if the local operator has re-run a given imaging acquisition stage multiple times, this may not be apparent simply from observing the current imaging device controller display, and so the remote expert may be unaware that the local operator is having difficulty. In such a situation, the local operator would preferably contact the remote expert for assistance, but the local operator may be reluctant to do so due to embarrassment, a desire to solve the problem independently, or the like.

US 2019/156484 A1 relates to improving imaging quality control, image processing, identification of findings in image data.

US 9 047 661 B2 relates to low-dose radiographing and and increasing image quality.

US 2019/261938 A1 relates to image quality ratings while performing imaging examination sessions.

EP 1 239 396 A2 relates to a touchscreen based a user interface for a medical diagnostic imaging device.

The following discloses certain improvements to overcome these problems and others.

### SUMMARY

In one aspect, a non-transitory computer readable medium stores instructions executable by at least one electronic processor to perform a method of providing remote monitoring of a local operator of a medical imaging device during a medical imaging examination. The method including: extracting image features from image frames displayed on a display device of a controller of the medical imaging device during medical imaging examinations performed using the medical imaging device; mapping the image features to medical imaging examinations based at least on timestamps of the image features, the medical imaging examinations to which the image features are mapped including completed medical imaging examinations and a current medical imaging examination; converting the image features mapped to each completed medical imaging examination into a representation of the completed medical imaging examination, wherein the representation of the completed medical imaging examination includes at least one completion time for the completed medical imaging examination; generating at least one completion time statistic representing the completion times for the completed medical imaging examinations or a subset of the completed medical imaging examinations; converting the image features mapped to the current medical imaging examination into a representation of a current status of the current medical imaging examination including at least an elapsed time of the current medical imaging examination; during the current medical imaging examination, displaying the representation of the current status of the current medical imaging examination on a workstation that is separate from the medical imaging device; and while displaying the representation of the current status of the current medical imaging examination on the workstation, additionally displaying a representation of the at least one completion time statistic on the workstation.

In another aspect, an apparatus for providing assistance from a remote expert to a local operator during a medical imaging examination performed using a medical imaging device includes a workstation operable by the remote expert. At least one electronic processor is programmed to: extract image features from image frames displayed on a display device of a controller of the medical imaging device during medical imaging examinations performed using the medical imaging device; map the image features to medical imaging examinations based at least on timestamps of the image features, the medical imaging examinations to which the image features are mapped including completed medical imaging examinations and a current medical imaging examination; convert the image features mapped to each completed medical imaging examination into a representation of the completed medical imaging examination, wherein the representation of the completed medical imaging examination includes at least one completion time for the completed medical imaging examination; generate at least one completion time statistic representing the completion times for the completed medical imaging examinations or a subset of the completed medical imaging examinations; converting the image features mapped to the current medical imaging examination into a representation of a current status of the current medical imaging examination including at least an elapsed time of the current medical imaging examination; during the current medical imaging examination, display the representation of the current status of the current medical imaging examination on a workstation that is separate from the medical imaging device; while displaying the representation of the current status of the current medical imaging examination on the workstation, additionally display a representation of the at least one completion time statistic on the workstation; and display an icon selectable by the remote expert to establish a communication pathway between the remote expert and the local operator.

In another aspect, a method of providing assistance from a remote expert to a local operator during a plurality of medical imaging examinations includes: extracting image features from image frames displayed on a display device of a controller of a plurality of medical imaging devices performing the plurality of medical imaging examinations; converting the image features mapped to the current medical imaging examination into a corresponding number of representations of a current status of each current medical imaging examination; and displaying each representation of the current status of each current medical imaging examination on a workstation that is separate from the medical imaging device.

One advantage resides in providing a remote expert or radiologist assisting a technician in conducting a medical imaging examination with situational awareness of local imaging examination(s) which facilitates providing effective assistance to one or more local operators at different facilities.

Another advantage resides in providing a remote expert or radiologist assisting one or more technicians in conducting a medical imaging examination with a list or other summary of relevant extracted information from shared screens of different medical imaging systems operated by technicians being assisted by the remote expert or radiologist.

Another advantage resides in providing a remote expert with a visualization of context-specific historical imaging examination data to allow the remote expert to determine whether to intervene in a current imaging examination.

Another advantage resides in a remote expert being able to provide assistance to a local technologist during an imaging examination without input from the local technologist.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.
FIGURE 1 diagrammatically shows an illustrative apparatus for providing remote assistance in accordance with the present disclosure.
FIGURE 2 shows an example flow chart of operations suitably performed by the apparatus of FIGURE 1.
FIGURES 3-8 show examples of an interface output generated by the apparatus of FIGURE 1.

### DETAILED DESCRIPTION

The following relates to a remote assistance system for assisting local imaging technicians in performing medical imaging examinations. Such a remote assistance system is sometimes referred to as a Radiology Operations Command Center (ROCC), and provides remote "supertech" assistance to a local technician performing an imaging examination. The ROCC may for example provide vendor- and model-agnostic screen sharing of the imaging device console with the remote expert (i.e., the "supertech"), along with videoconferencing capability. Other information feeds to the remote expert may also be provided, such as a bay camera for providing the remote expert with a view of the imaging bay preferably capturing the patient loading/unloading area and/or other critical area(s).

The ROCC preferably provides vendor- and model-agnostic screen sharing of the imaging device console with the remote expert, along with videoconferencing capability. Typically, an ROCC session is created by manual initiation of the ROCC session by the local technician. However, as previously noted, this initiative by the local technician may be hindered by various factors that may cause the local technician experiencing a problem to delay or even fail to proactively initiate an ROCC session. Such a delay or failure can lead to numerous adverse results such as degraded image quality, late-running imaging examinations that adversely impact the patient and the laboratory examinations schedule, and/or in some instances even equipment damage or harm to the patient (for example, if a PET scan needs to be rescheduled because the radiopharmaceutical has decayed to the point where a new dose must be administered - beside requiring a new examination, this increases the total radiation dose to the patient).

This following provides a more automated arrangement in which the remote expert can take a more proactive role in initiating an ROCC session. In one aspect, the remote expert is provided with an overview screen, for example a grid of icons, with each icon providing status information on a single monitored imaging bay. The icons may provide sufficient information for the remote expert to make a determination that assistance is needed (possibly after clicking on the icon to bring up additional real-time information on the current imaging examination), at which point the remote expert can initiate a videoconference. Additionally or alternatively, the system can automatically detect situations in which assistance may be needed, and provide an alert. In particular, the icons provide a (preferably graphical) summary of the examination time of the current medical examination (or some smaller unit such as a current stage of the current medical imaging examination) with information on a statistically expected completion time superimposed. In this way, the remote expert can immediately see whether the current examination is running overlong. Because various factors can impact the "expected" examination (or examination stage) completion time, in various embodiments the statistically expected completion time may be derived automatically from data collected by the ROCC on completed medical imaging examinations at whole-ROCC system level or at some smaller granularity (e.g., at the hospital level, or even for the specific local operator).

To implement the approach, the following discloses continually monitoring the shared imaging device display (even when that display is not being presented to the remote expert). This continuous monitoring suitably leverages the screen sharing hardware already in place for use during an ROCC assistance session. In some embodiments disclosed herein, the ROCC includes graphical user interface (GUI) screen templates that are used to extract information from the shared display, such as technologist ID, scan type, imaging device information, and current status of the imaging exam (e.g. which sequence is presently being performed). This information is extracted in real-time for the current imaging examination, but in embodiments disclosed herein also is recorded and stored to provide historical data on examination duration, individual imaging sequence (or other imaging stage) durations, and so forth. The mined data can be variously statistically analyzed, e.g. by technician, hospital, or so forth, and useful statistics such as average examination time and standard deviation of same can be extracted. This can be done dynamically (at higher computational cost) or on a weekly or other basis (at lower computational cost) and tabulated for the various types of imaging examinations, the various technologists, hospitals, or other desired granularities.

During the current imaging examination, the state of the current examination is compared against the average and/or standard deviation. Various contemplated ways of displaying this information are described herein, such as a time bar plotting the current examination progress and overlaying the average exam time and optionally also a standard deviation bar. Optionally, the state of the current examination can be compared automatically against the statistics and, if the current examination is taking unduly long (based on some threshold determined from the average time and possibly the standard deviation) then an audible and/or visual alert may be presented to the remote expert.

In some embodiments, the display format and/or alert behavior is dynamically adapted to the preferences of the remote expert, using reinforcement learning.

In addition to collecting historical data from the shared screen, in some embodiments additional information may be collected and utilized. For example, if the ROCC has authorized access to certain hospital databases via the HL-7 network then this could be mined, for example to determine patient age, gender, chronic conditions, or so forth. Such patient-specific information is then suitably used to adjust the average examination (or examination stage) time (e.g., statistics can be computed for patients with various chronic conditions such as obesity, COPD, or so forth) so that the average scan time used in the display/alerting is appropriate for the specific patient. Thus, for example, if the current examination is of an obese patient then the statistical average time for obese patients may be used, which may be longer than the statistical average time for all patients due to the increased difficulty in obese patient loading and positioning. Hence, the use of the patient-specific information can avoid issuing an erroneous alarm that the examination is running long.

In another example, if a bay camera is available then the imaging examination status monitoring can be extended to phases such as patient loading, patient unloading, and bay cleanup phases which are not reflected in the shared imaging display console. Thus, if the patient loading is taking unduly long the remote expert may be made aware of this.

It should be noted that the ROCC is not necessarily centralized at a single geographical location. In some embodiments, for example, the ROCC may comprise remote experts drawn from across an entire state, country, continent, or even drawn from across the world, and the ROCC is implemented as a distributed Internet-based infrastructure that provides data transfer (e.g. screen sharing and video feed transfer) and telephonic and/or video communication connectivity between the various experts and the imaging bays being assisted by those experts, and tracks time of the provided assistance, outcomes, and/or other metrics for billing or auditing purposes as may be called for in a given commercial implementation. Furthermore, in addition to the ROCC application, the disclosed systems and methods could find use in providing a central monitoring station for a larger medical institution or network. In such settings, the disclosed approach could be used to provide a radiology manager an overview of all imaging bays. Conversely, it is contemplated for the ROCC (more generally, assistance system) to have only a single remote expert on call at any given time.

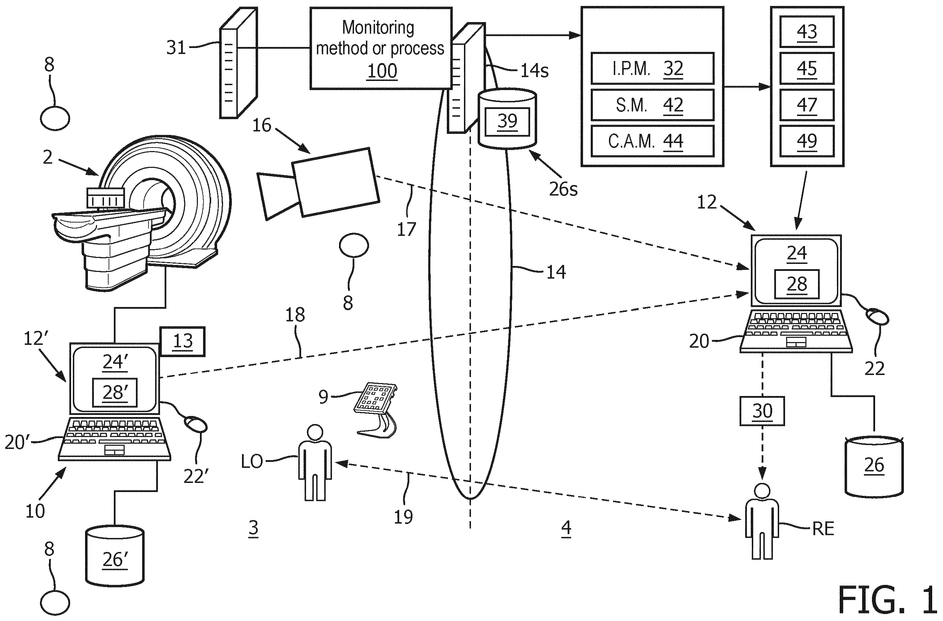

With reference to FIGURE 1, an apparatus for providing assistance from a remote medical imaging expert RE (or supertech) to a local technician operator LO is shown. As shown in FIGURE 1, the local operator LO, who operates one or more medical imaging devices (also referred to as an image acquisition device, imaging device, and so forth) 2, is located in a medical imaging device bay 3, and the remote operator RE is disposed in a remote service location or center 4. It should be noted that the "remote operator" RE may not necessarily directly operate the medical imaging device 2, but rather provides assistance to the local operator LO in the form of advice, guidance, instructions, or the like. The remote location 4 can be a remote service center, a radiologist's office, a radiology department, and so forth. The remote location 4 may be in the same building as the medical imaging device bay 3 (this may, for example, in the case of a "remote operator" RE who is a radiologist tasked with peri-examination image review), but more typically the remote service center 4 and the medical imaging device bay 3 are in different buildings, and indeed may be located in different cities, different countries, and/or different continents. In general, the remote location 4 is remote from the imaging device bay 3 in the sense that the remote operator RE cannot directly visually observe the imaging device 2 in the imaging device bay 3 (hence optionally providing a video feed or screen-sharing process as described further herein).

The image acquisition device 2 can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device 2 may also be a hybrid medical imaging device such as a PET/CT or SPECT/CT imaging system. While a single image acquisition device 2 is shown by way of illustration in FIGURE 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center 4 may provide service to multiple hospitals, and a single remote expert RE may concurrently monitor and provide assistance (when required) for multiple imaging bays being operated by multiple local operators, only one of which local operator is shown by way of representative illustration in FIGURE 1. The local operator controls the medical imaging device 2 via an imaging device controller 10. The remote operator is stationed at a remote workstation 12 (or, more generally, an electronic controller 12).

As used herein, the term "medical imaging device bay" (and variants thereof) refer to a room containing the medical imaging device 2 and also any adjacent control room containing the medical imaging device controller 10 for controlling the medical imaging device. For example, in reference to an MRI device, the medical imaging device bay 3 can include the radiofrequency (RF) shielded room containing the MRI device 2, as well as an adjacent control room housing the medical imaging device controller 10, as understood in the art of MRI devices and procedures. On the other hand, for other imaging modalities such as CT, the imaging device controller 10 may be located in the same room as the imaging device 2, so that there is no adjacent control room and the medical bay 3 is only the room containing the medical imaging device 2. In addition, while FIGURE 1 shows a single medical imaging device bay 3, it will be appreciated that the remote service center 4 (and more particularly the remote workstation 12) is in communication with multiple medical bays via a communication link 14, which typically comprises the Internet augmented by local area networks at the remote operator RE and local operator LO ends for electronic data communications.

A screen mirroring data stream 18 is generated by a screen sharing or capture device 13, and is sent from the imaging device controller 10 to the remote workstation 12. The screen mirroring data stream 18 is provided by a screen sharing or capture device 13, which in some embodiments is a DVI splitter, a HDMI splitter, and so forth that provides a split of the DVI feed from the medical imaging device controller 10 to an external display monitor of the medical imaging device controller 10. In other embodiments, the live video feed 17 may be provided by a video cable connecting an auxiliary video output (e.g. aux vid out) port of the imaging device controller 10 to the remote workstation 12 of the operated by the remote expert RE. In yet other embodiments, the screen sharing or capture device 13 is embodied by the medical imaging device controller 10 itself running screen-sharing software. The screen mirroring data stream 18 is sent to the remote workstation 12 via the communication link 14, e.g. as a streaming video feed received via a secure Internet link.

As diagrammatically shown in FIGURE 1, in some embodiments, a camera 16 (e.g., a video camera) is arranged to acquire a video stream 17 of a portion of the medical imaging device bay 3 that includes at least the area of the imaging device 2 where the local operator LO interacts with the patient, and optionally may further include the imaging device controller 10. The video stream 17 is also sent to the remote workstation 12 via the communication link 14, e.g. as a streaming video feed received via a secure Internet link.

The communication link 14 also provides a natural language communication pathway 19 for verbal and/or textual communication between the local operator and the remote operator. For example, the natural language communication link 19 may be a Voice-Over-Internet-Protocol (VOIP) telephonic connection, an online video chat link, a computerized instant messaging service, or so forth. Alternatively, the natural language communication pathway 19 may be provided by a dedicated communication link that is separate from the communication link 14 providing the data communications 17, 18, e.g. the natural language communication pathway 19 may be provided via a landline telephone. In another example, the natural language communication pathway 19 may be provided via an ROCC device 9, such as a mobile device (e.g., a tablet computer or a smartphone). For example, an "app" can run on the ROCC device 9 (operable by the local operator LO) and the remote workstation 12 (operable by the remote expert RE) to allow communication (e.g., audio chats, video chats, and so forth) between the local operator and the remote expert.

In some embodiments, one or more sensors 8 can additionally or alternatively be disposed in the medical imaging bay 3. The sensor(s) 8 are configured to collect data related to the events corresponding to the movement of the patient or medical personnel, in addition to the number of people, in the medical imaging bay 3. In one particular example, the sensor(s) 8 can include a radar sensor configured to detect persons in the medical imaging bay 3 containing the medical imaging device 2. The radar sensor could be in addition to, or in place of, the video camera 16.

FIGURE 1 also shows, in the remote service center 4 including the remote workstation 12, such as an electronic processing device, a workstation computer, or more generally a computer, which is operatively connected to receive and present the video 17 of the medical imaging device bay 3 from the camera 16 and to present the screen mirroring data stream 18 as a mirrored screen from the screen capture device 13. Additionally or alternatively, the remote workstation 12 can be embodied as a server computer or a plurality of server computers, e.g. interconnected to form a server cluster, cloud computing resource, or so forth. The workstation 12 includes typical components, such as an electronic processor 20 (e.g., a microprocessor), at least one user input device (e.g., a mouse, a keyboard, a trackball, and/or the like) 22, and at least one display device 24 (e.g. an LCD display, plasma display, cathode ray tube display, and/or so forth). In some embodiments, the display device 24 can be a separate component from the workstation 12. The display device 24 may also comprise two or more display devices, e.g. one display presenting the video 17 and the other display presenting the shared screen of the imaging device controller 10 generated from the screen mirroring data stream 18. Alternatively, the video and the shared screen may be presented on a single display in respective windows. The electronic processor 20 is operatively connected with a one or more non-transitory storage media 26. The non-transitory storage media 26 may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the workstation 12, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a graphical user interface (GUI) 28 for display on the remote operator display device 24.

The medical imaging device controller 10 in the medical imaging device bay 3 also includes similar components as the remote workstation 12 disposed in the remote service center 4. Except as otherwise indicated herein, features of the medical imaging device controller 10, which includes a local workstation 12', disposed in the medical imaging device bay 3 similar to those of the remote workstation 12 disposed in the remote service center 4 have a common reference number followed by a "prime" symbol, and the description of the components of the medical imaging device controller 10 will not be repeated. In particular, the medical imaging device controller 10 is configured to display a GUI 28' on a display device or controller display 24' that presents information pertaining to the control of the medical imaging device 2, such as configuration displays for adjusting configuration settings an alert 30 perceptible at the remote location when the status information on the medical imaging examination satisfies an alert criterion of the imaging device 2, imaging acquisition monitoring information, presentation of acquired medical images, and so forth. It will be appreciated that the screen mirroring data stream 18 carries the content presented on the display device **24'** of the medical imaging device controller 10. The communication link 14 allows for screen sharing between the display device 24 in the remote service center 4 and the display device 24' in the medical imaging device bay 3. The GUI 28' includes one or more dialog screens, including, for example, an examination/scan selection dialog screen, a scan settings dialog screen, an acquisition monitoring dialog screen, among others. The GUI 28' can be included in the video feed 17 or the mirroring data stream 18 and displayed on the remote workstation display 24 at the remote location **4.**

FIGURE 1 shows an illustrative local operator LO, and an illustrative remote expert RE (i.e. expert, e.g. supertech). However, in a Radiology Operations Command Center (ROCC) as contemplated herein, the ROCC provides a staff of supertechs who are available to assist a local operators LO at different hospitals, radiology labs, or the like. The ROCC may be housed in a single physical location, or may be geographically distributed. For example, in one contemplated implementation, the remote operators **RO** are recruited from across the United States and/or internationally in order to provide a staff of supertechs with a wide range of expertise in various imaging modalities and in various imaging procedures targeting various imaged anatomies. In view of this multiplicity of local operators **LO** and multiplicity of remote operators **RO,** the disclosed communication link **14** includes a server computer **14s** (or a cluster of servers, cloud computing resource comprising servers, or so forth) which is programmed to establish connections between selected local operator **LO**/remote expert **RE.** For example, if the server computer **14s** is Internet-based, then connecting a specific selected local operator **LO/**remote expert **RE** can be done using Internet Protocol (IP) addresses of the various components **16, 10, 12, 8, 9,** the telephonic or video terminals of the natural language communication pathway **19,** et cetera. The server computer **14s** is operatively connected with a one or more non-transitory storage media **26s.** The non-transitory storage media **26s** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer **14s,** various combinations thereof, or so forth. It is to be understood that any reference to a non-transitory medium or media **26s** herein is to be broadly construed as encompassing a single medium or multiple media of the same or different types. Likewise, the server computer **14s** may be embodied as a single electronic processor or as two or more electronic processors. The non-transitory storage media **26s** stores instructions executable by the server computer **14s.** In addition, the non-transitory computer readable medium **26s** (or another database) stores data related to a set of remote experts **RE** and/or a set of local operators **LO.** The remote expert data can include, for example, skill set data, work experience data, data related to ability to work on multi-vendor modalities, data related to experience with the local operator **LO** and so forth.

Furthermore, as disclosed herein the server **14s** performs a method or process **100** of providing remote monitoring of a local operator **LO** of the medical imaging device **2** during a medical imaging examination.

With continuing reference to FIGURE 1, in one embodiment of the method or process **100,** the server **14s** is programmed with several components to provide monitoring of the imaging examination, for use by remote expert **RE** during an assistance call, and also as disclosed herein to collect historical information on completed imaging examinations. An image processing module **32** is provided for processing image frames of the screen mirroring data stream **18** as a portion of a method or process **100** of providing assistance to the local operator during a medical imaging examination. The images (i.e. screen sharing image frames) are extracted from the screen mirroring data stream **18** received via the communication link **14.** The processing of the screen mirroring image frames by the image processing module **32** extracts relevant information such as an operator identifier that identifies the local operator **LO,** imaging examination workflow events such as the start and end of the imaging examination, the start and end of a particular stage of the imaging examination, and so forth. Other information may be extracted such as the make and model of the medical imaging device, the type of imaging examination being performed, patient information that is entered into the imaging device controller **10,** and/or so forth. It is noted that for the disclosed approach, this data collection is performed during each examination (or at least for many examinations) performed using the imaging device **2,** regardless of whether the local operator **LO** is requesting assistance from the remote expert **RE.** Even if the collected imaging examination information is not used for an assistance call, the collected information provides a database of historical data on completed imaging examinations from which completion time statistics are suitably derived. The various pieces of information extracted by the image processing module **32** are referred to herein as image features. The image processing module **32** also assigns timestamps to the image features based on the timestamps of the image frames (or groups of image frames) of the screen mirroring data stream **18** from which the image features are extracted.

A statistical modeler module **42** is configured to receive the image features extracted from the screen mirroring data stream **18** by the image processing module **32,** and optionally may also collect or retrieve historical data from a database **31** (e.g., an HL-7 database, an electronic medical record (EMR) database, an electronic health database (EHR), a health information system (HIS) database, a radiology information system (RIS) database, a digital imaging communication in medicine (DICOM) database, and so forth), the sensors **8,** and/or the video feed **17** acquired by the camera **16.** The statistical modeler module **34** is configured to map the image features extracted from the screen mirroring data stream **18** by the image processing module **32** to completed medical imaging examinations based at least on the timestamps of the image features, and to convert the image features mapped to each completed medical imaging examination into a representation **43** of the completed medical imaging examination. The representation of the completed medical imaging examination includes at least one completion time for the completed medical imaging examination. This may, for example, include a completion time for the completed medical imaging examination as a whole, and/or a completion time for one or more stages of the completed medical imaging examination. The statistical modeler module **42** then fits one or more statistical models to the completion times or other quantitative aspects of the representations of the completed medical imaging examinations. The statistical models may be applied to the entire set of completed medical imaging examinations, or so some subset defined by filters such as location and identification of the imaging device **2,** the type of imaging examination (e.g., "MR brain scan"), an identification of the local operator **LO,** patient demographics (e.g., age, gender, and so forth), and so forth. The resulting historical data can be represented as a distribution of values (e.g. imaging examination completion time (i.e. duration) in minutes, for the entire set of completed medical imaging examinations or for some subset such as a subset of technologists who have performed various exam types, etc.).

To map the image features to completed medical imaging examinations based at least on the timestamps of the image features, the statistical modeler module **42** is configured to use temporal matching algorithms to map the timestamps of the image features and/or timestamps of other collected information such as information from sensors **8** or the camera **16** to specific completed imaging examinations. This mapping can suitably use temporal matching algorithms operating on the timestamps to create clusters such that the features in the same cluster refer to the same imaging examination and features in different clusters refer to different entities. Temporal matching is well-suited to this task since successive imaging examinations are likely to be spaced apart in time by intervals during which the imaged patient is discharged and the next patient is admitted for an imaging examination. Hence, the timestamps are expected to form well-defined clusters spaced apart by discharge/admission time intervals. Temporal agreement/disagreement probability metrics are suitably used to perform this clustering operation. The statistical modeler module **42** is further configured to generate statistics on examination completion times or other quantitative examination metrics by producing point-statistic estimates (e.g., mean, median, min, max, variance, etc.) for the distributions from the historical data when appropriate.

A comparison and alerting module **44** is configured to compare a progression of the current imaging examination to the historical data, and determines if an alert **30** needs to be raised to the remote expert **RE.** The comparison and alerting module **44** optionally can account for patient context and the user context for determining when, how and which local operator **LO** to alert. This is suitably done by computing the completion time (or other competed examination) statistics for different subsets of the set of completed imaging examinations, e.g. the alerting module **44** may compare the current status of a current examination of an obese patient with the statistics generated by the statistical modeler module **42** for the subset of completed examinations performed on obese patients.

In some embodiments, the comparison and alerting module **34** is configured to compare the elapsed and remaining time of the current imaging examination to a simple threshold derived from the historical data (e.g. an upper quartile of the imaging examination time for the current imaging examination type for the performing local operator **LO)** and an alert **30** is raised if the total time is beyond this threshold.

In other embodiments, the comparison and alerting module **44** is configured to implement a machine learning algorithm and/or a reinforcement learning agent to learn from the available data and determine when to alert the remote expert **RE,** including feedback from the remote expert **RE.** This algorithm can learn the preferences of the remote expert **RE** on which data to display and how to display the data based on how often each available visualization is used to make sure the visualized data is actionable. In this embodiment, each intervention of the remote expert **RE** user to the local operator **LO** is a signal for the learning agent. Using a combination of thresholds from the historical data and interactions with the remote expert **RE,** the algorithm learns to adapt to the preferences of the remote expert **RE.** It would be rewarded when it causes the remote workstation **12** to displays the correct data on the right format and punished when it does not. For example, the algorithm can cause the remote workstation **12** to display a standard alert **30** when the elapsed and remaining time goes beyond the upper quartile of the historical data for this imaging examination for the local operator **LO.** The remote expert **RE** can dismiss the alert **30** when, for example, the remote expert **RE** checks the patient's age and realize that because patient is a child, and thus the imaging examination is expected to take longer than an imaging examination for an adult patient. This can be a signal for the reinforcement learning algorithm to not alert the remote expert **RE** the next time a patient with similar demographics is undergoing an imaging examination. In another example, the comparison and alerting module **34** can learn, for example, how the remote expert **RE** arranges different windows on the GUI **28,** and display the windows in this arrangement in the future.

The non-transitory computer readable medium **26s** of the server computer **14s** can store instructions executable by the server computer to perform the method **100** of providing remote monitoring of the local operator **LO** of the medical imaging device **2** during a medical imaging examination.

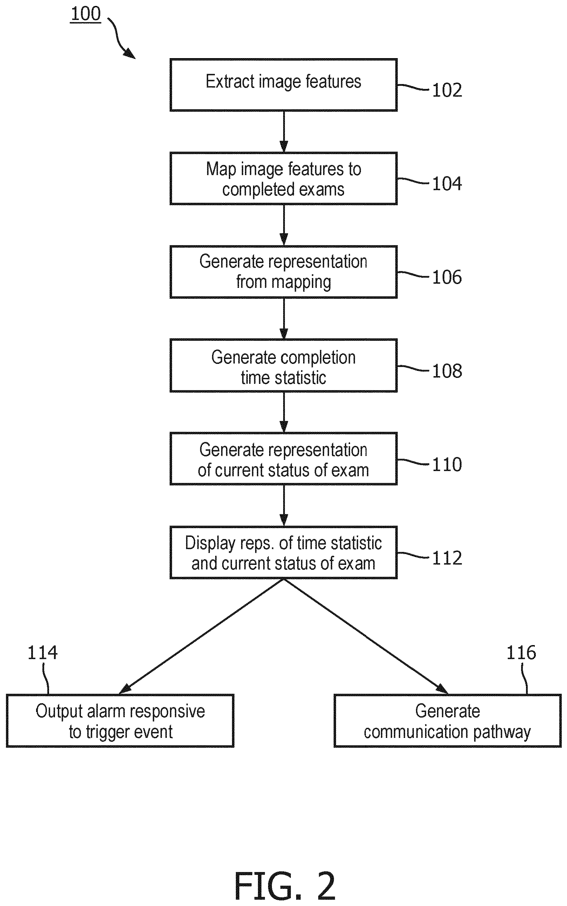

With reference to FIGURE 2, and with continuing reference to FIGURE 1, an illustrative embodiment of the monitoring method **100** is diagrammatically shown as a flowchart. To begin the monitoring method **100,** at an operation **102,** image features from image frames of the screen mirroring data stream **18** are extracted.

In one example, the image features can be extracted using the screen sharing device **13** (i.e., running screensharing software) of the medical imaging device controller **10** with the remote workstation **12.** In another example, the image features are extracted from the video feed **17** captured by the camera **16** and transmitted to the remote workstation **12.** This latter option is useful, for example, if monitoring a stage of the imaging examination such as patient loading in which the imaging device controller **10** is not involved (or at least is not heavily involved). The extracted information from the image features includes one or more of: an identification of the local operator **LO,** a modality of the medical imaging device **2,** a type of imaging examination being performed using the medical imaging device, and a progress of a current medical imaging examination, a completion time of the medical imaging examination, and/or an elapsed time of the imaging examination, along with a position of image features on the display device **24'** of the medical imaging device controller **10;** textual labels of the image features; a type of information of the image features; type of encoding of the image features; a type of formatting of the image features; a translation table or icon of the image features; and a shape or color of the image features, and so forth. In some examples, patient data can be retrieved from the database **31,** and features can be extracted from the patient data.

The extracting operation **102** can be performed in a variety of manners. In one example, the extraction includes performing an OCR process on the image frames to extract textual information. In another example, a corresponding dialog screen template **39** (stored in the non-transitory computer readable medium **26s** of the server computer **14s)** that corresponds to a dialog screen depicted in an image frame is identified. The corresponding dialog screen template **39** identifies one or more screen regions and associates the one or more screen regions with settings of the medical imaging examination. The extracted image features are extracted from the image frames and associated extracted information in the one or more screen regions with settings of the medical imaging examination using the associations provided by the corresponding dialog screen template **39.**

At an operation **104,** the extracted image features are mapped to medical imaging examinations based at least on timestamps of the image features. The medical imaging examinations to which the image features are mapped include completed medical imaging examinations and a current medical imaging examination. The mapping operation **104** can be performed by the statistical modeler module **32.** The mapping operation **104** includes mapping the extracted image features to specific imaging examinations based on the timestamps, for example using a temporal matching algorithm as previously described.

At an operation **106** also suitably performed by the statistical modeler module **42,** the image features mapped to each completed medical imaging examination are converted into a representation **43** of the completed medical imaging examination. The representation **43** of the completed medical imaging examination can include at least one completion time for the completed medical imaging examination, such as a completion time of the imaging examination as a whole, or completion times for various stages, sequences, or phases of the imaging examination.

At an operation **108** also performed by the statistical modeler module **42,** from this representation **43,** at least one completion time statistic **45** representing the completion times for the completed medical imaging examinations or a subset of the completed medical imaging examinations can be generated. These completion time statistics can include an average imaging examination completion time, and/or a standard deviation for the completion time. These could be for all completed examinations, or only for those completed examinations performed by the current local operator **LO.** In another embodiment, the at least one completion time for the completed medical imaging examination includes an examination completion time for the completed medical imaging examination. The at least one completion time statistic comprises an average examination completion time which is the average of the examination completion times of the completed medical examinations or the subset of the completed medical imaging examinations, and/or a standard deviation of the examination completion time which is the standard deviation of the examination completion times of the completed medical examinations or the subset of the completed medical imaging examinations.

The operations **102, 104** are suitably performed on an ongoing basis to collect image features for imaging examinations. The operations **106, 108** are performed to generate actionable statistics on completed imaging examinations. These operations **106, 108** may be performed in real-time to dynamically update the completed examination statistics as each examination is completed. However, such real-time dynamic updating can be computationally costly. In another approach, the operations **106, 108** are performed occasionally, e.g. once a week, once a month, or so forth.

The operations **110, 112, 114,** and **116** of FIGURE 2 are performed dynamically, in real-time during a current imaging examination. The operations **112, 114,** and **116** utilize the statistic(s) for completed imaging examinations generated by the operations **106, 108.** The most up-to-date statistics are preferably used, e.g. if the operations **106, 108** are performed once a month then the statistics generated in the most recent month are suitably used.

At the operation **110,** the image features mapped to the current medical imaging examination are converted into a representation **47** of a current status of the current medical imaging examination. The representation **47** includes at least an elapsed time of the current medical imaging examination, such as, for example, and elapsed time for the current imaging examination as a whole and/or an elapsed time for a current stage, sequence, or phase of the imaging examination.

The operations **106, 108,** and **110** can be performed in a variety of manners. In one example embodiment, the representation **43** of each completed medical imaging examination further includes an operator identifier that identifies the local operator **LO** who performed the completed medical imaging examination, and the representation **47** of the current status of the current medical imaging examination further includes a current operator identifier that identifies a local operator **LO** who is performing the current medical imaging examination. In this embodiment, the at least one completion time statistic **45** is generated for the subset of the completed medical imaging examinations whose operator identifiers match the current operator identifier.

In another example embodiment, the representation **43** of the completed medical imaging examination further includes a hospital identification that identifies a hospital where the completed medical imaging examination was performed, and the representation **47** of the current status of the current medical imaging examination further includes a current hospital identification that identifies a hospital where the current medical imaging examination is being performed. In this embodiment, the at least one completion time statistic **45** is generated for the subset of the completed medical imaging examinations whose hospital identification matches the current hospital identification.

In yet another example embodiment, the representation **47** of the current status of the current medical imaging examination includes a current stage of the current medical imaging examination and the elapsed time of the current imaging examination includes the elapsed time for the current stage of the completed medical imaging examination. The at least one completion time for the completed medical imaging examination includes an examination stage completion time corresponding to the current stage of the current medical imaging examination. The at least one completion time statistic **45** includes an average examination stage completion time which is the average of the examination stage completion times of the completed medical examinations or the subset of the completed medical imaging examinations, and/or a standard deviation of the examination stage completion time which is the standard deviation of the examination stage completion times of the completed medical examinations or the subset of the completed medical imaging examinations.

In another example embodiment, to generate the representation **43** of the completed medical imaging examination and the representation **47** of the current status of the current medical imaging examination, the extracted image features can be input into a generic imaging examination workflow model that is independent of a format of the image features displayed on the display device **24'** of the controller **10** operable by the local operator **LO.**

Once the representations **43, 45, 47** are generated, at an operation **112,** during the current medical imaging examination, the representation **45** of the current status of the current medical imaging examination on the workstation **12** during the imaging examination. That is, the representation **45** of the current status of the current medical imaging examination is displayed on a device separate from the medical imaging device **2.** While displaying the representation **43** of the current status of the current medical imaging examination on the workstation **12,** a representation of the at least one completion time statistic **45** is additionally displayed on the workstation **12.**

In one example embodiment of the displaying operation **112,** when the at least one completion time statistic **45** includes an average completion time, the displaying of the representation **47** of the current status of the current medical imaging examination on the workstation includes displaying a graphical representation of the elapsed time of the current medical imaging examination. In addition, the displaying of the representation of the at least one completion time statistic **45** includes displaying the average completion time as a graphical representation superimposed on the graphical representation of the elapsed time.

In another example embodiment, the at least one completion time statistic **45** includes a standard deviation of the completion time, and the displaying of the representation **47** of the current status of the current medical imaging examination further includes displaying the standard deviation of the completion time as a second graphical representation **49** superimposed on the graphical representation **47** of the elapsed time.

In other embodiments, the graphical representation of the elapsed time **47** comprises a time bar plot and the graphical representation of the average completion time **45** is displayed on or adjacent the time bar plot. In another example, the graphical representation of the elapsed time **47** comprises a time bar plot and the graphical representation **45** of the standard deviation is displayed on or adjacent the time bar plot.

These representations **43, 45, 47** can be displayed using a standard display format that is independent of the medical imaging device **2** operated by the local operator **LO** during the medical imaging examination.

In some embodiments, the method **100** includes an operation **114,** in which a trigger event can be identified based on a comparison of the elapsed time of the current medical imaging examination and the at least one completion time statistic **45,** at which an action needs to be taken by the remote expert **RE** and/or the local operator **LO.** An alert **30** indicating the trigger event can then be output via the GUI **28** of the remote workstation **12** (or be audibly output by the workstation **12).**

To provide the assistance functionality of the ROCC, the method **100** further includes an operation **116** via which the local operator **LO** is assisted by the remote expert **RE,** in which the communication pathway **19** is established between the local operator **LO** (via the ROCC service device **9)** and the remote expert **RE** (via the remote workstation **12).** To do so, a clickable icon **48** selectable by the remote expert **RE** can be displayed on the GUI **28** to initiate a videoconferencing call ore telephone call via the communication pathway **19** with the local operator **LO.** Advantageously, the remote expert **RE** may be prompted to do this based on an alarm output at the operation **114;** or may decide to initiate the call based on review of the display generated at operation **112** which provides a readily comprehended representation of the status of the current examination as compared with the statistical average completion time from the historical, i.e. completed, imaging examinations.

Although primarily described in terms of a single medical imaging device bay **3** housing a single medical imaging device **2,** the method **100** can be performed at a plurality of sites including medical imaging devices operated by a corresponding number of local operators, and the representations **43, 45, 47, 49** can include information from the sites of the plurality of sites. For example, the operation **102** can include obtaining images from a plurality of medical imaging devices **2** during multiple medical imaging examinations and extracting image features from image frames of the screen mirroring data stream **18.** The operation **110** can then include generating a metric, such as the representation **47,** indicative of a progression of each medical imaging examinations using the plurality of medical imaging devices **2.** The displaying operation **112** can include displaying the representation **47** for each medical imaging examination on the remote workstation **12** for the remote expert **RE** to monitor the progress of the imaging procedures.

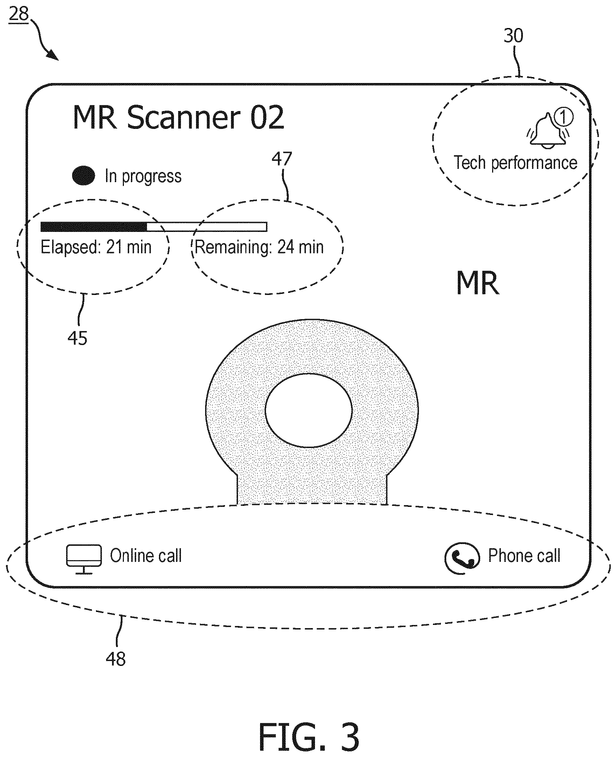

FIGURES 3-8 show different examples of the representations **43, 45, 47** as displayed on the GUI **28** of the remote workstation **12.** As shown in FIGURE 3, an alert **30** is shown (e.g. the bell indicator with the text "tech performance") to the remote expert **RE** whenever the elapsed and remaining time exceeds a given threshold (e.g. an upper quartile of the examination time for the current examination type for the performing local operator **LO,).** In another embodiment, the alert **30** can be raised by comparison and alerting module **44.** The remote expert **RE** can then click on the alert **30** to decide if an intervention is needed. The remote expert **RE** input will then become another signal for the learning agent of the comparison and alerting module **44.**

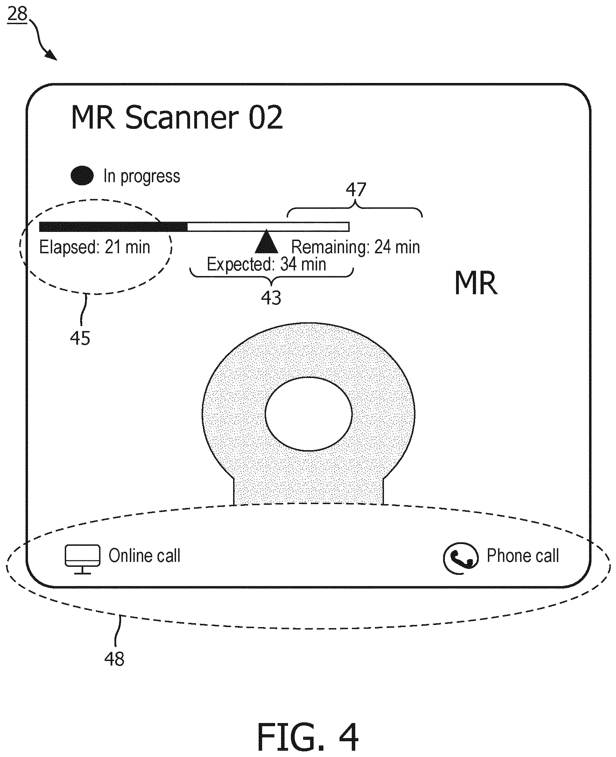

As shown in FIGURE 4, the representation **45** of the current medical examination is shown as an expected time of a current imaging examination displayed solely as a single-point estimation on top of a progress bar. The further the remaining time is from the expected value, the more likely that the examination is not going as planned and the more likely that there are reasons why the remote expert **RE** should intervene.

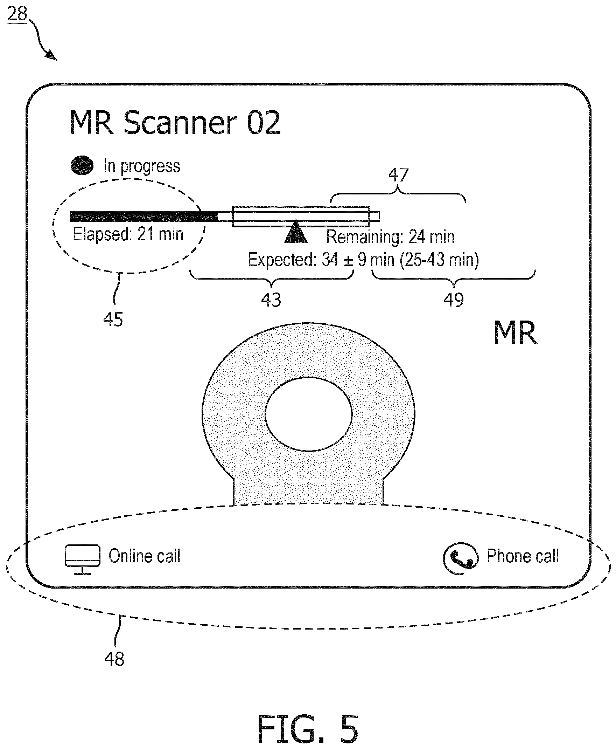

As shown in FIGURE 5, the representation **45** of the current medical examination is shown as an expected time of an imaging exam is displayed with a time-window of plus or minus ½ standard deviation as the second representation **49.** In this case, the farther the remaining time is from the expected time and the standard time window, the more likely that the examination is not going as planned and the more likely that there are reasons why the remote expert **RE** should intervene.

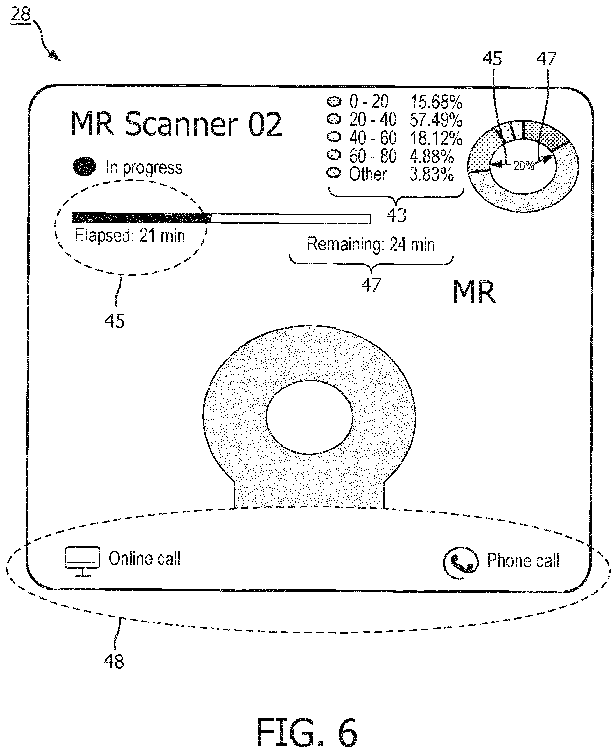

As shown in FIGURE 6, the representation **45** of the current medical examination is shown as a distribution of imaging examination duration as a pie chart, and the elapsed and remaining times representation **45, 47** are additionally displayed with arrows on top of the pie chart, similar to a clock. In this case, the 6 pm of the "clock" corresponds to the mean or median of the examination time duration distribution and whenever the remaining time indicator goes past the 6 pm mark, the more likely that the examination is not going as planned and the more likely that there are reasons why the remote expert **RE** should intervene. As shown in FIGURE 6, the examination takes anywhere from 20-40 minutes 57% of the time, and mean time is around 30 minutes. Since 21 minutes has already elapsed and the remaining time is still around 24 minutes, the remote expert **RE** might consider intervening.

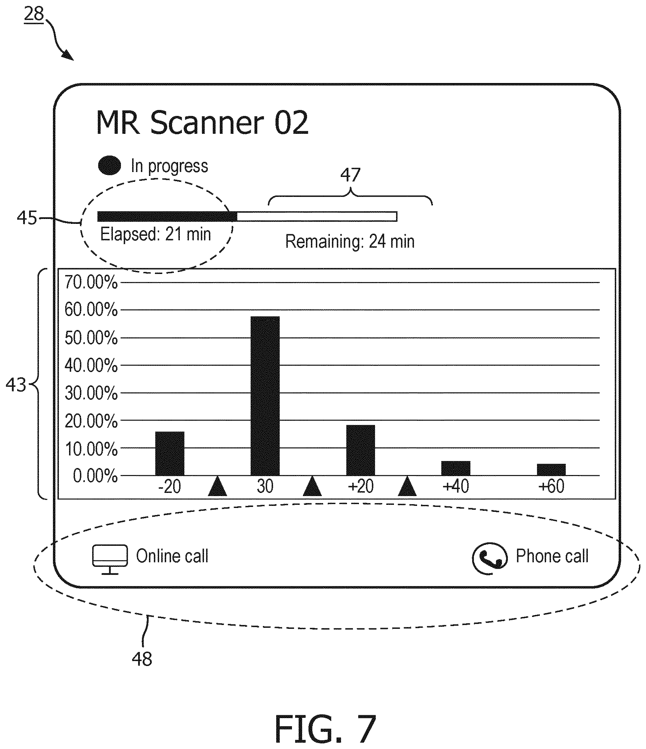

As shown in FIGURE 7, the representation **45** of the current medical examination is shown as distribution of time represented as a histogram with the most expected time represented on the x-axis in the middle and then the tail ends of the distribution are represented relatively, by adding and subtracting minutes. A y-axis shows the percentage of times the examinations have completed in that time frame. The elapsed time in the current examination, remaining time in the current examination and the scheduled arrival of the next patient are represented on the distribution using different markers. In this embodiment, if the remaining time marker is too close to the next patient's arrival, or if the elapsed and remaining time markers are falling too close to the right side of the distribution, the more likely that the examination is not going as expected, and has the potential to disrupt downstream workflow and the more likely remote expert **RE** might have a reason to intervene.

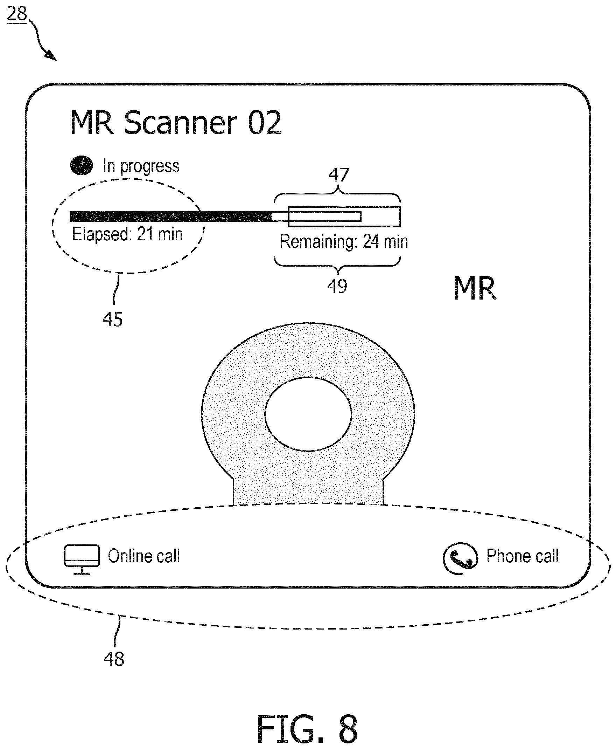

As shown in FIGURE 8, the representation **45** of the current medical examination is shown as a progression of different sequences of the examination. A standard-deviation time window **49** is overlaid on the progress bar **47** that displays the progression of sequences. There are three sequences that needs to be completed, two of them are completed so far, and the local operator **LO** is imaging the third sequence currently, which falls within the expected time window from the historical data.

FIGURES 3-8 show various representations of the time statistic and current status of the current imaging examination generated by the operation **112** and displayed on the GUI **28.** These representations of FIGURES 3-8 are for a single current imaging examination. However, it is to be appreciated that in a scaled-up ROCC, the remote expert **RE** may be charged with monitoring a number of different imaging examinations that are being performed concurrently in different imaging bays (which, in turn, may in some embodiments be located in different cities, different states, or even different countries). To enable the remote expert **RE** to keep track of all these concurrent imaging examinations, in some embodiments the GUI **28** provides a grid display, with one representation of the type shown in FIGURES 3-8 being depicted for each imaging examination. Hence, for example, if the remote expert **RE** is charged with monitoring 12 imaging examinations that are being performed concurrently, then the grid will include 12 representation corresponding to the 12 imaging examinations, with each representation being suitably formulated as one of the examples of FIGURES 3-8. In this way, the remote expert **RE** can quickly, at a glance, assess the status of each current imaging examination as compared against the statistical completion time for that type of imaging examination; and can thereby quickly identify if any imaging examination seems to be experiencing a problem as indicated by its elapsed time exceeding the statistical completion time. This enables the remote expert **RE** to take proactive action, such as initiating a videoconference with the local operator **LO** performing the imaging examination that is experiencing a problem. Similarly, the alarm operation **114** can provide the prompt for the remote expert **RE** to initiate the videoconference.

The disclosure has been described with reference to the preferred embodiments. Modifications and alterations may occur to others upon reading and understanding the preceding detailed description. The scope of protection is defined by the claims.

## Claims

1. A method **(100)** of providing remote monitoring of a local operator **(LO)** of a medical imaging device **(2)** during a medical imaging examination, the method comprises the steps of:
extracting image features from image frames displayed on a display device **(24')** of a controller **(10)** of the medical imaging device during medical imaging examinations performed using the medical imaging device;
mapping the image features to medical imaging examinations based at least on timestamps of the image features, the medical imaging examinations to which the image features are mapped including completed medical imaging examinations and a current medical imaging examination;
converting the image features mapped to each completed medical imaging examination into a representation **(43)** of the completed medical imaging examination, wherein the representation of the completed medical imaging examination includes at least one completion time for the completed medical imaging examination;
generating at least one completion time statistic **(45)** representing the completion times for the completed medical imaging examinations or a subset of the completed medical imaging examinations;
converting the image features mapped to the current medical imaging examination into a representation **(47)** of a current status of the current medical imaging examination including at least an elapsed time of the current medical imaging examination;
during the current medical imaging examination, displaying the representation of the current status of the current medical imaging examination on a workstation **(12)** that is separate from the medical imaging device; and
while displaying the representation of the current status of the current medical imaging examination on the workstation, displaying a representation of the at least one completion time statistic on the workstation.

2. The method of claim 1, wherein:
the representation **(43)** of the completed medical imaging examination further includes an operator identifier that identifies the local operator **(LO)** who performed the completed medical imaging examination;
the representation **(47)** of the current status of the current medical imaging examination further includes a current operator identifier that identifies a local operator who is performing the current medical imaging examination; and
the at least one completion time statistic **(45)** is generated for the subset of the completed medical imaging examinations whose operator identifiers match the current operator identifier or
the representation **(43)** of the completed medical imaging examination further includes a hospital identification that identifies a hospital where the completed medical imaging examination was performed;
the representation **(47)** of the current status of the current medical imaging examination further includes a current hospital identification that identifies a hospital where the current medical imaging examination is being performed; and
the at least one completion time statistic **(45)** is generated for the subset of the completed medical imaging examinations whose hospital identification matches the current hospital identification.

3. The method of claim 1, wherein:
the at least one completion time for the completed medical imaging examination includes an examination completion time for the completed medical imaging examination, and the at least one completion time statistic **(45)** comprises one or more of:
an average examination completion time which is the average of the examination completion times of the completed medical examinations or the subset of the completed medical imaging examinations; and
a standard deviation of the examination completion time which is the standard deviation of the examination completion times of the completed medical examinations or the subset of the completed medical imaging examinations; or
the representation **(47)** of the current status of the current medical imaging examination includes a current stage of the current medical imaging examination and the elapsed time of the current imaging examination includes the elapsed time for the current stage of the completed medical imaging examination,
the at least one completion time statistic **(45)** for the completed medical imaging examination includes an examination stage completion time corresponding to the current stage of the current medical imaging examination; and
the at least one completion time statistic comprises one or more of:
an average examination stage completion time which is the average of the examination stage completion times of the completed medical examinations or the subset of the completed medical imaging examinations; and
a standard deviation of the examination stage completion time which is the standard deviation of the examination stage completion times of the completed medical examinations or the subset of the completed medical imaging examinations.

4. The method of any one of claims 1-3, wherein:
the at least one completion time statistic **(45)** includes an average completion time;
the displaying of the representation **(47)** of the current status of the current medical imaging examination on the workstation includes displaying a graphical representation of the elapsed time of the current medical imaging examination; and
the displaying of the representation of the at least one completion time statistic on the workstation includes displaying the average completion time as a graphical representation superimposed on the graphical representation of the elapsed time.

5. The method of claim 4, wherein:
the at least one completion time statistic **(45)** includes a standard deviation of the completion time, and
the displaying of the representation **(47)** of the current status of the current medical imaging examination on the workstation **(12)** further includes displaying the standard deviation of the completion time as a second graphical representation **(49)** superimposed on the graphical representation of the elapsed time.

6. The method of claim 4, wherein the graphical representation **(47)** of the elapsed time comprises a time bar plot, the graphical representation **(45)** of the average completion time is displayed on or adjacent the time bar plot, and, in particular, the graphical representation **(47)** of the elapsed time comprises a time bar plot and the graphical representation **(49)** of the standard deviation is displayed on or adjacent the time bar plot.

7. The method of any one of claims 1-6, wherein the method **(100)** further includes the steps of:
identifying a trigger event based on a comparison of the elapsed time of the current medical imaging examination and the at least one completion time statistic **(45);** and
causing the workstation **(12)** to output an audible and/or visual alert **(30)** in response to identifying the trigger event.

8. The method of any one of claims 1-7, wherein extracting image features from image frames displayed on the display device **(24')** of the controller **(10)** operable by the local operator **(LO)** during the medical imaging examination further includes:
identifying a corresponding dialog screen template **(39)** that corresponds to a dialog screen depicted in an image frame wherein the corresponding dialog screen template identifies one or more screen regions and associates the one or more screen regions with settings of the medical imaging examination; and
extracting information from the image frame and associating the extracted information in the one or more screen regions with settings of the medical imaging examination using the associations provided by the corresponding dialog screen template.

9. The method according to any one of claims 1-8, wherein the method **(100)** further includes:
extracting the image features from the image frames displayed on the display device **(24')** of the controller **(10)** using screen sharing software running on the controller or
at the workstation **(12)** operated by the remote expert **(RE),** receiving a video feed **(17)** capturing the display device **(24')** of the controller **(10)** operated by the local operator **(LO);**
displaying the video feed at the workstation operated by the remote expert; and
extracting the image features from the received video feed.

10. The method according to any one of claims 1-9, wherein
the method **(100)** further includes displaying the representation **(43)** at the workstation **(12)** operated by the remote expert **(RE)** using a standard display format that is independent of the medical imaging device **(2)** operated by the local operator **(LO)** during the medical imaging examination; and/or
the method **(100)** is performed at a plurality of sites including medical imaging devices **(2)** operated by a corresponding number of local operators **(LO),** and the representation **(43)** include information from the sites of the plurality of sites.

11. The method of any one of claims 1-10, wherein the method **(100)** further includes establishing a communication pathway between the remote expert **(RE)** and the local operator **(LO)** by displaying an icon **(48)** selectable by the remote expert on the remote workstation **(12)** to initiate a videoconferencing call with the local operator.

12. An apparatus **(10)** for providing assistance from a remote expert **(RE)** to a local operator **(LO)** during a medical imaging examination performed using a medical imaging device **(2),** the apparatus comprises:
a workstation **(12)** operable by the remote expert; and
at least one electronic processor **(14s)** programmed to:
extract image features from image frames displayed on a display device **(24')** of a controller **(10)** of the medical imaging device during medical imaging examinations performed using the medical imaging device;
map the image features to medical imaging examinations based at least on timestamps of the image features, the medical imaging examinations to which the image features are mapped including completed medical imaging examinations and a current medical imaging examination;
convert the image features mapped to each completed medical imaging examination into a representation **(43)** of the completed medical imaging examination, wherein the representation of the completed medical imaging examination includes at least one completion time for the completed medical imaging examination;
generate at least one completion time statistic **(45)** representing the completion times for the completed medical imaging examinations or a subset of the completed medical imaging examinations;
converting the image features mapped to the current medical imaging examination into a representation **(47)** of a current status of the current medical imaging examination including at least an elapsed time of the current medical imaging examination;
during the current medical imaging examination, display the representation of the current status of the current medical imaging examination on a workstation **(12)** that is separate from the medical imaging device;
while displaying the representation of the current status of the current medical imaging examination on the workstation, display a representation of the at least one completion time statistic on the workstation; and
display an icon **(48)** selectable by the remote expert to establish a communication pathway between the remote expert and the local operator.

13. The apparatus **(10)** of claim 12, wherein the at least one completion time for the completed medical imaging examination includes an examination completion time for the completed medical imaging examination, and the at least one completion time statistic **(45)** comprises one or more of:
an average examination completion time which is the average of the examination completion times of the completed medical examinations or the subset of the completed medical imaging examinations; and
a standard deviation of the examination completion time which is the standard deviation of the examination completion times of the completed medical examinations or the subset of the completed medical imaging examinations.

14. A computer-readable storage medium comprising instructions which, when executed by a computer, cause the computer to carry out the steps of the method of any one of claims 1-11.

15. A computer program comprising instructions which, when the program is executed by a computer, cause the computer to carry out the steps of the method of any one of claims 1-11.

## Patentansprüche

1. Verfahren (100) zum Bereitstellen einer Fernüberwachung eines lokalen Bedieners (LO) einer medizinischen Bildgebungsvorrichtung (2) während einer bildgebenden medizinischen Untersuchung, wobei das Verfahren die folgenden Schritte umfasst:
Extrahieren von Bildmerkmalen aus Bildframes, die auf einer Anzeigevorrichtung (24') einer Steuerung (10) der medizinischen Bildgebungsvorrichtung während bildgebender medizinischer Untersuchungen angezeigt werden, die unter Verwendung der medizinischen Bildgebungsvorrichtung durchgeführt werden;
Abbilden der Bildmerkmale auf bildgebende medizinische Untersuchungen basierend zumindest auf Zeitstempeln der Bildmerkmale, wobei die bildgebenden medizinischen Untersuchungen, auf die die Bildmerkmale abgebildet sind, abgeschlossene bildgebende medizinische Untersuchungen und eine aktuelle bildgebende medizinische Untersuchung beinhalten;
Umwandeln der Bildmerkmale, die auf jede abgeschlossene bildgebende medizinische Untersuchung abgebildet sind, in eine Repräsentation (43) der abgeschlossenen bildgebenden medizinischen Untersuchung, wobei die Repräsentation der abgeschlossenen bildgebenden medizinischen Untersuchung mindestens eine Abschlusszeit für die abgeschlossene bildgebende medizinische Untersuchung beinhaltet;
Erzeugen mindestens einer Abschlusszeitstatistik (45), die die Abschlusszeiten für die abgeschlossenen bildgebenden medizinischen Untersuchungen oder eine Teilmenge der abgeschlossenen bildgebenden medizinischen Untersuchungen repräsentiert;
Umwandeln der Bildmerkmale, die auf die aktuelle bildgebende medizinische Untersuchung abgebildet sind, in eine Repräsentation (47) eines aktuellen Status der aktuellen bildgebenden medizinischen Untersuchung, einschließlich zumindest einer verstrichenen Zeit der aktuellen bildgebenden medizinischen Untersuchung;
während der aktuellen bildgebenden medizinischen Untersuchung, Anzeigen der Repräsentation des aktuellen Status der aktuellen bildgebenden medizinischen Untersuchung auf einer Arbeitsstation (12), die von der medizinischen Bildgebungsvorrichtung getrennt ist; und
während des Anzeigens der Repräsentation des aktuellen Status der aktuellen bildgebenden medizinischen Untersuchung auf der Workstation, Anzeigen einer Repräsentation der mindestens einen Abschlusszeitstatistik auf der Arbeitsstation.

2. Verfahren nach Anspruch 1, wobei:
die Repräsentation (43) der abgeschlossenen bildgebenden medizinischen Untersuchung ferner eine Bedienerkennung beinhaltet, die den lokalen Bediener (LO) identifiziert, der die abgeschlossene bildgebende medizinische Untersuchung durchgeführt hat;
die Repräsentation (47) des aktuellen Status der aktuellen bildgebenden medizinischen Untersuchung ferner eine aktuelle Bedienerkennung beinhaltet, die einen lokalen Bediener identifiziert, der die aktuelle bildgebende medizinische Untersuchung durchführt; und
die mindestens eine Abschlusszeitstatistik (45) für die Teilmenge der abgeschlossenen bildgebenden medizinischen Untersuchungen erzeugt wird, deren Bedienerkennungen mit der aktuellen Bedienerkennung übereinstimmen, oder
die Repräsentation (43) der abgeschlossenen bildgebenden medizinischen Untersuchung ferner eine Krankenhausidentifikation beinhaltet, die ein Krankenhaus identifiziert, in dem die abgeschlossene bildgebende medizinische Untersuchung durchgeführt wurde;
die Repräsentation (47) des aktuellen Status der aktuellen bildgebenden medizinischen Untersuchung ferner eine aktuelle Krankenhausidentifikation beinhaltet, die ein Krankenhaus identifiziert, in dem die aktuelle bildgebende medizinische Untersuchung durchgeführt wird; und
die mindestens eine Abschlusszeitstatistik (45) für die Teilmenge der abgeschlossenen bildgebenden medizinischen Untersuchungen erzeugt wird, deren Krankenhausidentifikation mit der aktuellen Krankenhausidentifikation übereinstimmt.

3. Verfahren nach Anspruch 1, wobei:
die mindestens eine Abschlusszeit für die abgeschlossene bildgebende medizinische Untersuchung eine Untersuchungsabschlusszeit für die abgeschlossene bildgebende medizinische Untersuchung beinhaltet und die mindestens eine Abschlusszeitstatistik (45) eines oder mehrere von Folgendem umfasst:
eine durchschnittliche Untersuchungsabschlusszeit, die der Durchschnitt der Untersuchungsabschlusszeiten der abgeschlossenen medizinischen Untersuchungen oder der Teilmenge der abgeschlossenen bildgebenden medizinischen Untersuchungen ist; und
eine Standardabweichung der Untersuchungsabschlusszeit, die die Standardabweichung der Untersuchungsabschlusszeiten der abgeschlossenen medizinischen Untersuchungen oder der Teilmenge der abgeschlossenen bildgebenden medizinischen Untersuchungen ist; oder
die Repräsentation (47) des aktuellen Status der aktuellen bildgebenden medizinischen Untersuchung einen aktuellen Abschnitt der aktuellen bildgebenden medizinischen Untersuchung beinhaltet und die verstrichene Zeit der aktuellen bildgebenden Untersuchung die verstrichene Zeit für den aktuellen Abschnitt der abgeschlossenen bildgebenden medizinischen Untersuchung beinhaltet,
die mindestens eine Abschlusszeitstatistik (45) für die abgeschlossene bildgebende medizinische Untersuchung eine Untersuchungsabschnittsabschlusszeit beinhaltet, die dem aktuellen Abschnitt der aktuellen bildgebenden medizinischen Untersuchung entspricht; und
die mindestens eine Abschlusszeitstatistik eines oder mehrere von Folgendem umfasst:
eine durchschnittliche Untersuchungsabschnittsabschlusszeit, die der Durchschnitt der Untersuchungsabschnittsabschlusszeiten der abgeschlossenen medizinischen Untersuchungen oder der Teilmenge der abgeschlossenen bildgebenden medizinischen Untersuchungen ist; und
eine Standardabweichung der Untersuchungsabschnittsabschlusszeit, die die Standardabweichung der Untersuchungsabschnittsabschlusszeiten der abgeschlossenen medizinischen Untersuchungen oder der Teilmenge der abgeschlossenen bildgebenden medizinischen Untersuchungen ist.

4. Verfahren nach einem der Ansprüche 1-3, wobei:
die mindestens eine Abschlusszeitstatistik (45) eine durchschnittliche Abschlusszeit beinhaltet;
das Anzeigen der Repräsentation (47) des aktuellen Status der aktuellen bildgebenden medizinischen Untersuchung auf der Arbeitsstation das Anzeigen einer grafischen Repräsentation der verstrichenen Zeit der aktuellen bildgebenden medizinischen Untersuchung beinhaltet; und
das Anzeigen der Repräsentation der mindestens einen Abschlusszeitstatistik auf der Arbeitsstation das Anzeigen der durchschnittlichen Abschlusszeit als eine grafische Repräsentation überlagert auf die grafische Repräsentation der verstrichenen Zeit beinhaltet.

5. Verfahren nach Anspruch 4, wobei:
die mindestens eine Abschlusszeitstatistik (45) eine Standardabweichung der Abschlusszeit beinhaltet, und
das Anzeigen der Repräsentation (47) des aktuellen Status der aktuellen bildgebenden medizinischen Untersuchung auf der Arbeitsstation (12) ferner das Anzeigen der Standardabweichung der Abschlusszeit als eine zweite grafische Repräsentation (49) überlagert auf die grafische Repräsentation der verstrichenen Zeit beinhaltet.

6. Verfahren nach Anspruch 4, wobei die grafische Repräsentation (47) der verstrichenen Zeit eine Zeitbalkendarstellung umfasst, die grafische Repräsentation (45) der durchschnittlichen Abschlusszeit auf oder neben der Zeitbalkendarstellung angezeigt wird und insbesondere die grafische Repräsentation (47) der verstrichenen Zeit eine Zeitbalkendarstellung umfasst und die grafische Repräsentation (49) der Standardabweichung auf oder neben der Zeitbalkendarstellung angezeigt wird.

7. Verfahren nach einem der Ansprüche 1-6, wobei das Verfahren (100) ferner die folgenden Schritte beinhaltet:
Identifizieren eines Auslöseereignisses basierend auf einem Vergleich der verstrichenen Zeit der aktuellen bildgebenden medizinischen Untersuchung und der mindestens einen Abschlusszeitstatistik (45); und
Bewirken als Reaktion auf das Identifizieren des Auslöseereignisses, dass die Arbeitsstation (12) eine akustische und/oder visuelle Warnung (30) ausgibt.

8. Verfahren nach einem der Ansprüche 1-7, wobei das Extrahieren von Bildmerkmalen aus Bildframes, die auf der Anzeigevorrichtung (24') der Steuerung (10) angezeigt werden, die durch den lokalen Bediener (LO) während der bildgebenden medizinischen Untersuchung betreibbar ist, ferner Folgendes beinhaltet:
Identifizieren einer entsprechenden Dialogbildschirmvorlage (39), die einem Dialogbildschirm entspricht, der in einem Bildframe dargestellt ist, wobei die entsprechende Dialogbildschirmvorlage einen oder mehrere Bildschirmbereiche identifiziert und den einen oder die mehreren Bildschirmbereiche mit Einstellungen der bildgebenden medizinischen Untersuchung assoziiert; und
Extrahieren von Informationen aus dem Bildframe und Assoziieren der extrahierten Informationen in dem einen oder den mehreren Bildschirmbereichen mit Einstellungen der bildgebenden medizinischen Untersuchung unter Verwendung der Assoziationen, die durch die entsprechende Dialogbildschirmvorlage bereitgestellt werden.

9. Verfahren nach einem der Ansprüche 1-8, wobei das Verfahren (100) ferner Folgendes beinhaltet:
Extrahieren der Bildmerkmale aus den Bildframes, die auf der Anzeigevorrichtung (24') der Steuerung (10) unter Verwendung von Bildschirmfreigabesoftware angezeigt werden, die auf der Steuerung ausgeführt wird, oder
an der Arbeitsstation (12), die von dem entfernten Experten (RE) betrieben wird, Empfangen eines Videofeed (17), der die Anzeigevorrichtung (24') der Steuerung (10) erfasst, die von dem lokalen Bediener (LO) betrieben wird;
Anzeigen des Videofeed an der Arbeitsstation, die vom entfernten Experten betrieben wird; und
Extrahieren der Bildmerkmale aus dem empfangenen Videofeed.

10. Verfahren nach einem der Ansprüche 1-9, wobei
das Verfahren (100) ferner Anzeigen der Repräsentation (43) an der Arbeitsstation (12) beinhaltet, die durch den entfernten Experten (RE) betrieben wird, unter Verwendung eines Standardanzeigeformats, das unabhängig von der medizinischen Bildgebungsvorrichtung (2) ist, die durch den lokalen Bediener (LO) während der bildgebenden medizinischen Untersuchung betrieben wird; und/oder
das Verfahren (100) an mehreren Standorten durchgeführt wird, einschließlich medizinischer Bildgebungsvorrichtungen (2), die durch eine entsprechende Anzahl von lokalen Bedienern (LO) betrieben werden, und die Repräsentation (43) Informationen von den Standorten der mehreren Standorte beinhaltet.

11. Verfahren nach einem der Ansprüche 1-10, wobei das Verfahren (100) ferner das Einrichten eines Kommunikationspfades zwischen dem entfernten Experten (RE) und dem lokalen Bediener (LO) durch Anzeigen eines Icons (48) beinhaltet, das durch den entfernten Experten auf der entfernten Arbeitsstation (12) auswählbar ist, um einen Videokonferenzanruf mit dem lokalen Bediener zu initiieren.

12. Einrichtung (10) zum Bereitstellen von Unterstützung von einem entfernten Experten (RE) für einen lokalen Bediener (LO) während einer bildgebenden medizinischen Untersuchung, die unter Verwendung einer medizinischen Bildgebungsvorrichtung (2) durchgeführt wird, wobei die Einrichtung Folgendes umfasst:
eine Arbeitsstation (12), die durch den entfernten Experten betreibbar ist; und
mindestens einen elektronischen Prozessor (14s), der programmiert ist zum:
Extrahieren von Bildmerkmalen aus Bildframes, die auf einer Anzeigevorrichtung (24') einer Steuerung (10) der medizinischen Bildgebungsvorrichtung während bildgebender medizinischer Untersuchungen angezeigt werden, die unter Verwendung der medizinischen Bildgebungsvorrichtung durchgeführt werden;
Abbilden der Bildmerkmale auf bildgebende medizinische Untersuchungen basierend zumindest auf Zeitstempeln der Bildmerkmale, wobei die bildgebenden medizinischen Untersuchungen, auf die die Bildmerkmale abgebildet sind, abgeschlossene bildgebende medizinische Untersuchungen und eine aktuelle bildgebende medizinische Untersuchung beinhalten;
Umwandeln der Bildmerkmale, die auf jede abgeschlossene bildgebende medizinische Untersuchung abgebildet sind, in eine Repräsentation (43) der abgeschlossenen bildgebenden medizinischen Untersuchung, wobei die Repräsentation der abgeschlossenen bildgebenden medizinischen Untersuchung mindestens eine Abschlusszeit für die abgeschlossene bildgebende medizinische Untersuchung beinhaltet;
Erzeugen mindestens einer Abschlusszeitstatistik (45), die die Abschlusszeiten für die abgeschlossenen bildgebenden medizinischen Untersuchungen oder eine Teilmenge der abgeschlossenen bildgebenden medizinischen Untersuchungen repräsentiert;
Umwandeln der Bildmerkmale, die auf die aktuelle bildgebende medizinische Untersuchung abgebildet sind, in eine Repräsentation (47) eines aktuellen Status der aktuellen bildgebenden medizinischen Untersuchung, einschließlich zumindest einer verstrichenen Zeit der aktuellen bildgebenden medizinischen Untersuchung;
während der aktuellen bildgebenden medizinischen Untersuchung, Anzeigen der Repräsentation des aktuellen Status der aktuellen bildgebenden medizinischen Untersuchung auf einer Arbeitsstation (12), die von der medizinischen Bildgebungsvorrichtung getrennt ist;
während des Anzeigens der Repräsentation des aktuellen Status der aktuellen bildgebenden medizinischen Untersuchung auf der Workstation, Anzeigen einer Repräsentation der mindestens einen Abschlusszeitstatistik auf der Arbeitsstation; und
Anzeigen eines Icons (48), das vom entfernten Experten ausgewählt werden kann, um einen Kommunikationspfad zwischen dem entfernten Experten und dem lokalen Bediener einzurichten.

13. Einrichtung (10) nach Anspruch 12, wobei die mindestens eine Abschlusszeit für die abgeschlossene bildgebende medizinische Untersuchung eine Untersuchungsabschlusszeit für die abgeschlossene bildgebende medizinische Untersuchung beinhaltet und die mindestens eine Abschlusszeitstatistik (45) eines oder mehrere von Folgendem umfasst:
eine durchschnittliche Untersuchungsabschlusszeit, die der Durchschnitt der Untersuchungsabschlusszeiten der abgeschlossenen medizinischen Untersuchungen oder der Teilmenge der abgeschlossenen bildgebenden medizinischen Untersuchungen ist; und
eine Standardabweichung der Untersuchungsabschlusszeit, die die Standardabweichung der Untersuchungsabschlusszeiten der abgeschlossenen medizinischen Untersuchungen oder der Teilmenge der abgeschlossenen bildgebenden medizinischen Untersuchungen ist.

14. Computerlesbares Speichermedium, das Anweisungen umfasst, die, wenn sie durch einen Computer ausgeführt werden, bewirken, dass der Computer die Schritte des Verfahrens nach einem der Ansprüche 1-11 ausführt.

15. Computerprogramm, das Anweisungen umfasst, die, wenn das Programm durch einen Computer ausgeführt wird, bewirken, dass der Computer die Schritte des Verfahrens nach einem der Ansprüche 1-11 ausführt.

## Revendications

1. Procédé (100) permettant de fournir une surveillance à distance d'un opérateur local (LO) d'un dispositif d'imagerie médicale (2) pendant un examen d'imagerie médicale, le procédé comprend les étapes suivantes :
l'extraction de caractéristiques d'image à partir d'images affichées sur un dispositif d'affichage (24') d'un contrôleur (10) du dispositif d'imagerie médicale pendant des examens d'imagerie médicale effectués à l'aide du dispositif d'imagerie médicale ;
le mise en correspondance des caractéristiques d'image avec des examens d'imagerie médicale sur la base au moins d'horodatages des caractéristiques d'image, les examens d'imagerie médicale avec lesquels les caractéristiques d'image sont mises en correspondance incluant des examens d'imagerie médicale terminés et un examen d'imagerie médicale en cours ;
la conversion des caractéristiques d'image mises en correspondance avec chaque examen d'imagerie médicale terminé en une représentation (43) de l'examen d'imagerie médicale terminé, la représentation de l'examen d'imagerie médicale terminé incluant au moins un temps d'exécution de l'examen d'imagerie médicale terminé ;
la génération d'au moins une statistique de temps d'exécution (45) représentant les temps d'exécution pour les examens d'imagerie médicale terminés ou un sous-ensemble des examens d'imagerie médicale terminés ;
la conversion des caractéristiques d'image mises en correspondance avec l'examen d'imagerie médicale en cours en une représentation (47) d'un état actuel de l'examen d'imagerie médicale en cours incluant au moins un temps écoulé de l'examen d'imagerie médicale en cours ;
pendant l'examen d'imagerie médicale en cours, l'affichage de la représentation de l'état actuel de l'examen d'imagerie médicale en cours sur un poste de travail (12) qui est séparé du dispositif d'imagerie médicale ; et
pendant l'affichage de la représentation de l'état actuel de l'examen d'imagerie médicale en cours sur le poste de travail, l'affichage d'une représentation de l'au moins une statistique de temps d'exécution sur le poste de travail.

2. Procédé selon la revendication 1, dans lequel :
la représentation (43) de l'examen d'imagerie médicale terminé inclut en outre un identifiant d'opérateur qui identifie l'opérateur local (LO) qui a effectué l'examen d'imagerie médicale terminé ;
la représentation (47) de l'état actuel de l'examen d'imagerie médicale en cours inclut en outre un identifiant d'opérateur actuel qui identifie un opérateur local qui effectue l'examen d'imagerie médicale en cours ; et
l'au moins une statistique de temps d'exécution (45) est générée pour le sous-ensemble des examens d'imagerie médicale terminés dont les identifiants d'opérateur correspondent à l'identifiant d'opérateur actuel ou
la représentation (43) de l'examen d'imagerie médicale terminé inclut en outre une identification d'hôpital qui identifie un hôpital où l'examen d'imagerie médicale terminé a été effectué ;
la représentation (47) de l'état actuel de l'examen d'imagerie médicale en cours inclut en outre une identification d'hôpital actuel qui identifie un hôpital où l'examen d'imagerie médicale en cours est effectué ; et
l'au moins une statistique de temps d'exécution (45) est générée pour le sous-ensemble des examens d'imagerie médicale terminés dont l'identification de l'hôpital correspond à l'identification d'hôpital actuel.

3. Procédé selon la revendication 1, dans lequel :
l'au moins un temps d'exécution de l'examen d'imagerie médicale terminé inclut un temps d'exécution d'examen pour l'examen d'imagerie médicale terminé, et l'au moins une statistique de temps d'exécution (45) comprend un ou plusieurs des éléments suivants :
un temps moyen d'exécution d'examen qui est la moyenne des temps d'exécution d'examen des examens médicaux terminés ou du sous-ensemble des examens d'imagerie médicale terminés ; et
un écart type du temps d'exécution d'examen qui est l'écart type des temps d'exécution d'examen des examens médicaux terminés ou du sous-ensemble des examens d'imagerie médicale terminés ; ou
la représentation (47) de l'état actuel de l'examen d'imagerie médicale en cours inclut un stade actuel de l'examen d'imagerie médicale en cours et le temps écoulé de l'examen d'imagerie en cours inclut le temps écoulé pour le stade actuel de l'examen d'imagerie médicale terminé,
l'au moins une statistique de temps d'exécution (45) de l'examen d'imagerie médicale terminé inclut un temps d'exécution de stade d'examen correspondant au stade actuel de l'examen d'imagerie médicale en cours ; et
l'au moins une statistique de temps d'exécution comprend un ou plusieurs des éléments suivants :
un temps d'exécution moyen de stade d'examen qui est la moyenne des temps d'exécution de stade d'examen des examens médicaux terminés ou du sous-ensemble des examens d'imagerie médicale terminés ; et
un écart type du temps d'exécution de stade d'examen, qui est l'écart type des temps d'exécution de stage d'examen des examens médicaux terminés ou du sous-ensemble des examens d'imagerie médicale terminés.

4. Procédé selon l'une quelconque des revendications 1 à 3, dans lequel :
l'au moins une statistique de temps d'exécution (45) inclut un temps d'exécution moyen ;
l'affichage de la représentation (47) de l'état actuel de l'examen d'imagerie médicale en cours sur le poste de travail inclut l'affichage d'une représentation graphique du temps écoulé de l'examen d'imagerie médicale en cours ; et
l'affichage de la représentation de l'au moins une statistique de temps d'exécution sur le poste de travail inclut l'affichage du temps d'exécution moyen sous forme de représentation graphique superposée à la représentation graphique du temps écoulé.

5. Procédé selon la revendication 4, dans lequel :
l'au moins une statistique de temps d'exécution (45) inclut un écart type du temps d'exécution, et
l'affichage de la représentation (47) de l'état actuel de l'examen d'imagerie médicale en cours sur le poste de travail (12) comporte en outre l'affichage de l'écart type du temps d'exécution sous forme d'une seconde représentation graphique (49) superposée à la représentation graphique du temps écoulé.

6. Procédé selon la revendication 4, dans lequel la représentation graphique (47) du temps écoulé comprend un graphique à barres temporel, la représentation graphique (45) du temps d'exécution moyen est affichée sur ou à côté du graphique à barres temporel, et, en particulier, la représentation graphique (47) du temps écoulé comprend un graphique à barres temporel et la représentation graphique (49) de l'écart type est affichée sur ou à côté du graphique à barres temporel.

7. Procédé selon l'une quelconque des revendications 1 à 6, dans lequel le procédé (100) comporte en outre les étapes suivantes :
l'identification d'un événement déclencheur sur la base d'une comparaison du temps écoulé de l'examen d'imagerie médicale en cours et de l'au moins une statistique de temps d'exécution (45) ; et
la commande du poste de travail (12) pour qu'il produise une alerte sonore et/ou visuelle (30) en réponse à l'identification de l'événement déclencheur.

8. Procédé selon l'une quelconque des revendications 1 à 7, dans lequel l'extraction de caractéristiques d'image à partir d'images affichées sur le dispositif d'affichage (24') du contrôleur (10) pouvant être opérée par l'opérateur local (LO) pendant l'examen d'imagerie médicale comporte en outre :
l'identification d'un modèle d'écran de dialogue correspondant (39) qui correspond à un écran de dialogue représenté dans une image où le modèle d'écran de dialogue correspondant identifie une ou plusieurs régions d'écran et associe les une ou plusieurs régions d'écran à des paramètres de l'examen d'imagerie médicale ; et
l'extraction d'informations à partir de l'image et l'association des informations extraites dans les une ou plusieurs régions d'écran à des paramètres de l'examen d'imagerie médicale à l'aide des associations fournies par le modèle d'écran de dialogue correspondant.

9. Procédé selon l'une quelconque des revendications 1 à 8, dans lequel le procédé (100) comporte en outre :
l'extraction des caractéristiques d'image à partir d'images affichées sur le dispositif d'affichage (24') du contrôleur (10) à l'aide d'un logiciel de partage d'écran exécuté sur le contrôleur ou
au niveau du poste de travail (12) manipulé par l'expert distant (RE), la réception d'un flux vidéo (17) capturant le dispositif d'affichage (24') du contrôleur (10) manipulé par l'opérateur local (LO) ;
l'affichage du flux vidéo sur le poste de travail manipulé par l'expert distant ; et
l'extraction des caractéristiques d'image du flux vidéo reçu.

10. Procédé selon l'une quelconque des revendications 1 à 9, dans lequel
le procédé (100) comporte en outre l'affichage de la représentation (43) au niveau du poste de travail (12) manipulé par l'expert distant (RE) à l'aide d'un format d'affichage standard indépendant du dispositif d'imagerie médicale (2) manipulé par l'opérateur local (LO) pendant l'examen d'imagerie médicale ; et/ou
le procédé (100) est mis en œuvre sur une pluralité de sites comportant des dispositifs d'imagerie médicale (2) manipulés par un nombre correspondant d'opérateurs locaux (LO), et la représentation (43) inclut des informations provenant des sites de la pluralité de sites.

11. Procédé selon l'une quelconque des revendications 1 à 10, dans lequel le procédé (100) comporte en outre l'établissement d'une voie de communication entre l'expert distant (RE) et l'opérateur local (LO) en affichant une icône (48) sélectionnable par l'expert distant sur le poste de travail distant (12) pour initier un appel en visioconférence avec l'opérateur local.

12. Appareil (10) permettant de fournir l'assistance d'un expert distant (RE) à un opérateur local (LO) pendant un examen d'imagerie médicale effectué au moyen d'un dispositif d'imagerie médicale (2), l'appareil comprenant :
un poste de travail (12) manipulable par l'expert distant ; et
au moins un processeur électronique (14s) programmé pour :
extraire des caractéristiques d'image à partir d'images affichées sur un dispositif d'affichage (24') d'un contrôleur (10) du dispositif d'imagerie médicale pendant des examens d'imagerie médicale effectués à l'aide du dispositif d'imagerie médicale ;
mettre en correspondance des caractéristiques d'image avec des examens d'imagerie médicale sur la base au moins d'horodatages des caractéristiques d'image, les examens d'imagerie médicale avec lesquels les caractéristiques d'image sont mises en correspondance incluant des examens d'imagerie médicale terminés et un examen d'imagerie médicale en cours ;
convertir les caractéristiques d'image mises en correspondance avec chaque examen d'imagerie médicale terminé en une représentation (43) de l'examen d'imagerie médicale terminé, la représentation de l'examen d'imagerie médicale terminé incluant au moins un temps d'exécution de l'examen d'imagerie médicale terminé ;
générer au moins une statistique de temps d'exécution (45) représentant les temps d'exécution pour les examens d'imagerie médicale terminés ou un sous-ensemble des examens d'imagerie médicale terminés ;
convertir les caractéristiques d'image mises en correspondance avec l'examen d'imagerie médicale en cours en une représentation (47) d'un état actuel de l'examen d'imagerie médicale en cours incluant au moins un temps écoulé de l'examen d'imagerie médicale en cours ;
pendant l'examen d'imagerie médicale en cours, afficher la représentation de l'état actuel de l'examen d'imagerie médicale en cours sur un poste de travail (12) qui est séparé du dispositif d'imagerie médicale ;
pendant l'affichage de la représentation de l'état actuel de l'examen d'imagerie médicale en cours sur le poste de travail, afficher une représentation de l'au moins une statistique de temps d'exécution sur le poste de travail ; et
afficher une icône (48) sélectionnable par l'expert distant pour établir une voie de communication entre l'expert distant et l'opérateur local.

13. Appareil (10) selon la revendication 12, dans lequel l'au moins un temps d'exécution de l'examen d'imagerie médicale terminé inclut un temps d'exécution d'examen pour l'examen d'imagerie médicale terminé, et l'au moins une statistique de temps d'exécution (45) comprend un ou plusieurs des éléments suivants :
un temps moyen d'exécution d'examen qui est la moyenne des temps d'exécution d'examen des examens médicaux terminés ou du sous-ensemble des examens d'imagerie médicale terminés ; et
un écart type du temps d'exécution d'examen qui est l'écart type des temps d'exécution d'examen des examens médicaux terminés ou du sous-ensemble des examens d'imagerie médicale terminés.

14. Support de stockage lisible par ordinateur comprenant des instructions qui, lorsqu'elles sont exécutées par un ordinateur, amènent l'ordinateur à exécuter les étapes du procédé selon l'une quelconque des revendications 1 à 11.

15. Programme informatique comprenant des instructions qui, lorsque le programme est exécuté par un ordinateur, amènent l'ordinateur à exécuter les étapes du procédé selon l'une quelconque des revendications 1 à 11.
